# EUROPEAN PATENT SPECIFICATION

(11) **EP 0 779 821 B1**
(45) Date of publication and mention of the grant of the patent: **27.03.2002**
(21) Application number: 95930653.1
(22) Date of filing: 06.09.1995
(51) Int. Cl.: A61K 49/00, A61M 31/00, A61F 2/30, A61K 9/00

(54) **IMPROVEMENTS IN OR RELATING TO CONTRAST AGENTS**
VERBESSERUNGEN IN DEN ODER IN BEZUG ZU KONTRASTMITTELN
AMELIORATIONS APPORTEES A DES AGENTS DE CONTRASTE

(30) Priority: 06.09.1994 GB 9417941
(43) Date of publication of application: 25.06.1997
(73) Proprietor: NYCOMED IMAGING AS, 0401 Oslo (NO)
(72) Inventor: KLAVENESS, Jo, N-1166 Oslo (NO); RONGVED, Pal, N-1450 Nesoddtangen (NO); STRANDE, Per, N-0755 Oslo (NO)
(74) Representative: Canning, Lewis R.
(86) International application number: PCT/GB95/02109
(87) International publication number: WO 96/07434

(56) References cited:
- WO-A-92/04392
- WO-A-92/17212
- WO-A-93/17718
- WO-A-95/06518
- WO-A-95/21631

## Description

This invention relates to novel contrast agents, more particularly to new gas-containing polymer-based contrast agents of use in diagnostic imaging, and to the novel polymer components thereof.

Published International Patent Application No. WO 93/17718, the contents of which are incorporated herein by reference, discloses contrast agents comprising gas-containing or gas-generating polymer microparticles and/or microballoons characterised in that the polymer is a biodegradable polymer containing units of formula

- (O)ₘ-CO-O-C(R¹R²) -O-CO- (O)ₙ- (I)

(wherein R¹ and R² each represent a hydrogen atom or a carbon-attached monovalent organic group or R¹ and R² together form a carbon-attached divalent organic group, and m and n are each zero or 1). Such contrast agents, which may be used in diagnostic applications such as ultrasound and MR imaging, exhibit good storage stability coupled with good stability and contrast effect in vivo following administration, often for several passages of circulation. The contrast agents are, however, thereafter readily biodegradable in vivo by virtue of the susceptibility of the units of formula (I) to degradation by common esterase enzymes.

Biodegradable polymers containing units of formula (I) as above are described in WO 92/04392, where it is stated that particles of such polymers containing ultrasound contrast agents such as heavy materials, e.g. barium sulphate or iodinated compounds, may be used as ultrasound contrast media.

WO 92/17212 describes ultrasound contrast agents comprising microbubbles of gas or a gas precursor encapsulated by non-proteinaceous crosslinked or polymerised amphiphilic moieties. The encapsulating material may contain biodegradable crosslinking groups which may contain units of formula (I) as above.

WO 95/06518 describes polymer-based gas-containing contrast agents in which microbubbles of gas are encapsulated by non-polymerisable wall-forming block or graft copolymer surfactants. The copolymer surfactants are preferably biodegradable, and may contain units of formula (I) as above.

WO 95/21631 describes microparticulate contrast agents comprising gas or a gas precursor encapsulated by non-polymeric and non-polymerisable wall-forming material which may advantageously contain units of formula (I) as above.

The present invention concerns contrast agents which fall within the overall scope of the above-mentioned WO 93/17718 but which are not specifically disclosed thereby. This novel class of contrast agents is particularly advantageous by virtue of the agents' excellent stability and contrast effect in vivo and the fact that the agents degrade in the body to products which are well-tolerated and in most cases are endogenous.

According to one aspect of the present invention there are provided contrast agents comprising gas-containing polymer microparticles and/or microballoons characterised in that the polymer is a biodegradable polymer consisting of repeating units of formula (II) 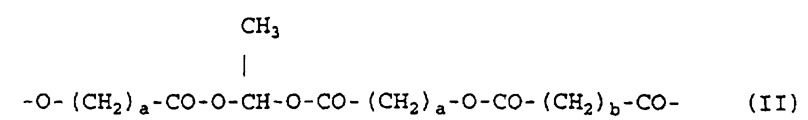 (where a represents an integer in the range 9-19, e.g. 13-17, and b represents an integer in the range 1-8, e.g. 3-6). Such contrast agents have been found to exhibit very sharp ultrasound contrast effects in animal tests, for example providing both myocardial contrast enhancement in dogs in all parts of the ventricular wall and excellent contrast enhancement of the kidney. Echogenicity may also be retained following uptake of the contrast agents by the reticuloendothelial system, permitting use as a macrophage imaging agent.

The contrast agents of the invention exhibit excellent storage stability, for example maintaining their echogenicity in an aqueous suspension for eight weeks at 25°C. They are, however, rapidly degraded and eliminated from the body following administration, e.g. having a half life of 1-2 days in the liver.

It will be appreciated that the principal in vivo degradation products of polymers comprising repeating units of formula (II) will be ω-hydroxyacids of formula HO.(CH₂)ₐ.COOH (where a is as hereinbefore defined), diacids of formula HOOC.(CH₂)_{b}.COOH (where b is as hereinbefore defined) and acetaldehyde. Acetaldehyde is an endogenous substance which will be oxidised in vivo to acetic acid, as in the metabolism of ethanol. The integers a and b may advantageously be chosen so as to generate endogenous ω-hydroxyacids and diacids; thus, for example, polymers in which a=15 and b=4 will degrade to yield 16-hydroxypalmitic acid and adipic acid, both of which are endogenous.

The contrast agents of the invention may be used in a variety of diagnostic imaging techniques, including ultrasound, MR and X-ray imaging. Their use in diagnostic ultrasonic imaging and in MR imaging, e.g. as susceptibility contrast agents, constitute preferred features of the invention.

Any biocompatible gas may be employed in the contrast agents of the invention, for example air, nitrogen, oxygen, hydrogen, nitrous oxide, carbon dioxide, helium, argon, sulphur hexafluoride, low molecular weight optionally fluorinated hydrocarbons such as methane, acetylene, carbon tetrafluoride and other perfluoroalkanes such as perfluoropropane, perfluorobutane and perfluoropentane, and mixtures of any of the foregoing. The gas may be free within the microbubble or may be trapped or entrained within a containing substance. The term "gas" as used herein includes any substances in gaseous (including vapour) form at 37°C.

For ultrasonic applications such as echocardiography, in order to permit free passage through the pulmonary system and to achieve resonance with the preferred imaging frequency of about 0.1-15 MHz, it may be convenient to employ microbubbles having an average size of 0.1-10 µm, e.g. 1-7 µm. Substantially larger bubbles, e.g. with average sizes of up to 500 µm, may however be useful in other applications, for example gastrointestinal imaging or investigations of the uterus or Fallopian tubes.

The contrast agents of the invention may incorporate additives such as emulsifying agents, coating agents, plasticisers, bulking agents, cryoprotectants and/or antioxidants, for example to modify their stability, dispersibility, aggregation tendencies, biological properties etc., or to modify the flexibility and/or polarity of the membrane.

Representative emulsifying agents include fatty acids (e.g. straight chain saturated or unsaturated fatty acids, for example containing 10-20 carbon atoms) and carbohydrate and triglyceride esters thereof; proteins such as gelatin or, more preferably, human serum albumin; phospholipids, e.g. lecithin; polysaccharides such as starch, modified (e.g. lipophilised) starch or gum arabic; and surface active polymers such as polyvinyl alcohols, polyethylene glycols and block copolymers (including extended polymers), for example poly(oxyethylene)-poly(oxypropylene)-poly(oxyethylene) block copolymers such as Pluronics.

Where block copolymer (including extended polymer) surfactants are employed, these may contain biodegradable linkages of formula (I) as hereinbefore defined, for example in which R¹ and R² (when other than hydrogen) may each represent a carbon-attached hydrocarbyl or heterocyclic group, for example having up to 20 carbon atoms, e.g. an aliphatic group such as an alkyl or alkenyl group (preferably having up to 10 carbon atoms), a cycloalkyl group (preferably having up to 10 carbon atoms), an araliphatic group such as an aralkyl group (preferably having up to 20 carbon atoms), an aryl group (preferably having up to 20 carbon atoms) or a heterocyclic group having up to 20 carbon atoms and one or more heteroatoms selected from O, S and N. Such a hydrocarbyl or heterocyclic grouping may carry one or more functional groups such as halogen atoms or groups of the formulae -NR³R⁴, -CONR³R⁴, -OR⁵, -SR⁵ and -COOR⁶, where R³ and R⁴ are each hydrogen atoms, acyl groups or hydrocarbyl groups as defined for R¹ and R²; R⁵ is a hydrogen atom, an acyl group or a group as defined for R¹ or R²; and R⁶ is a hydrogen atom or a group as defined for R¹ or R². Where R¹ and R² represent a divalent grouping this may, for example, be an alkylidene, alkenylidene, alkylene or alkenylene group (preferably having up to 10 carbon atoms), which may carry one or more functional groups as defined above.

The presence in such block copolymers of units of formula (I) in which R¹ and R² are selected from hydrogen atoms and methyl groups, e.g. in which R¹ represents a hydrogen atom and R² represents a methyl group, may be advantageous; it may also be advantageous to select units in which m and n are zero.

The block copolymer surfactant may comprise two or more blocks of differing lyophilicity, for example in linear di-block, tri-block or multi-block arrays, e.g. of the type A-B, A-B-A, B-A-B or A-B-A-B-A where A and B are polymer blocks of differing lyophilicity, preferably being hydrophilic and hydrophobic blocks respectively. Branched structures, e.g. of the type 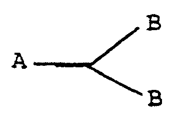 and macrocyclic structures, e.g. of the type 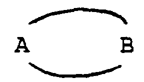 may also be employed.

Hydrophilic blocks in such block copolymer surfactants may, for example, be derived from polymers such as polysaccharides, polyalcohols (e.g. polyvinyl alcohol), polyvinylpyrrolidones, polyethylene glycols and poly(amino acids). Polymers such as polyorthoesters, polyacetals, polyanhydrides, polyglycolic acids, poly(meth)acrylic acids and derivatives such as esters thereof, substituted as necessary by hydrophilic groups, may also be useful. The hydrophilic blocks may advantageously consist essentially of polyethylene glycol units.

Hydrophobic blocks in such block copolymer surfactants may, for example, be derived from oil-soluble condensation, ionic and free-radical generated polymers, for example poly(meth)acrylate esters, polyorthoesters, vinylic and styrenic polymers, polyacetals, polyanhydrides, polyglycolic acids and ethers and esters thereof, and polylactic acid/ polyglycolic acid copolymers; such polymers may, for example, incorporate or be substituted with hydrophobic groups such as alkyl, aralkyl or aryl groups to increase their hydrophobicity. The hydrophobic blocks may advantageously comprise a polyester chain containing one or more long chain aliphatic groups (e.g. C₁₀₋₂₀ polymethylene groups) linked by and/or incorporating units of formula (I). Such hydrophobic blocks may be oligomeric or quasi-polymeric, as in extended polymers, and as such may include monomeric groups, which may for example exhibit polymer characteristics (e.g. as a result of the presence of long chain units) while not stricly possessing a definable repeating unit.

One preferred class of block copolymer surfactants thus comprises copolymers containing polyethylene glycol units as the hydrophilic blocks and units of formula

-R^{a}-(CH₂)ₐ-CO-O-CH(CH₃)-O-CO-(CH₂)ₐ-R^{b}- (III)

(where a is as hereinbefore defined and R^{a} and R^{b} each represent valence bonds or linking groups such as carbonyl groups or diacid residues of formula -O-CO-(CH₂)_{b}-CO-O where b is as hereinbefore defined) in the hydrophobic part, as an extending moiety or as a moiety in an oligomeric or polymeric block.

Other preferred classes of surfactants include fatty acid acylated polyethylene glycols such as MYRJ® s and extended polymers comprising a methoxy-terminated polyethylene glycol hydrophilic block acylated with a hydrophobic moiety comprising a chain of two or more fatty acids, for example an acyloxyacyl group such as 16-hexadecanoyloxyhexadecanoyl.

Representative bulking agents and cryoprotectants include alcohols, for example aliphatic alcohols such as t-butanol, polyols such as glycerol, sugars such as sucrose, mannitol, trehalose or cyclodextrins, and polyglycols such as polyethylene glycol.

Representative preservatives include antioxidants.

The contrast agents of the invention may be prepared in a number of ways, e.g. as described in WO 93/17718, for example by incorporation of a gas into a biodegradable polymer comprising repeating units of formula (II) so as to form polymer microparticles and/or microballoons.

One useful method corresponds to the interfacial deposition techniques described in EP-A-0398935 and EP-A-0458745 and comprises dissolving or suspending the polymer in a water-immiscible organic solvent, emulsifying (e.g. by high speed stirring or high shear mixing) the resulting solution or suspension in an aqueous phase, preferably in the presence of a surfactant to stabilise the resulting oil-in-water emulsion, and subsequently removing at least the organic phase, preferably both phases (e.g. by evaporation or lyophilisation, preferably under an atmosphere of the gas which is to be incorporated, e.g. under reduced pressure) whereby the polymer forms a membrane at the interface between the aqueous and organic phases.

Organic solvents useful in such processes include aliphatic, cycloaliphatic and araliphatic hydrocarbons, e.g. containing up to 10 carbon atoms, for example n-octane, cyclooctane, cyclohexane, a dimethylcyclohexane, ethylcyclohexane, a methylheptane, an ethylhexane, toluene, xylene or a terpene, terpenoid or isoprenoid such as camphene or limonene; haloalkanes, such as dichloromethane, chloroform, carbon tetrachloride, methyl bromide or a Freon; esters, such as ethyl or propyl acetate, butyl formate or propyl or isopropyl butyrate or isobutyrate; and appropriate ethers and other lipophilic solvents. Solvents such as camphene are of advantage in that they are biotolerated, so that it is not necessary to remove all solvent residues from the contrast agent prior to administration. Such high-melting solvents may also be advantageous in processes in which the emulsion is frozen and lyophilised, since they will rapidly solidify under these conditions and so may enhance the structural integrity of the resulting microparticulate contrast agent.

As noted above, the emulsifying procedure is preferably effected in the presence of a surfactant. Such emulsifying agents and/or any other additives, e.g. as hereinbefore described, may conveniently be predissolved in the aqueous phase.

Prior to phase removal it may be advantageous to subject the emulsion to filtration and/or extrusion, e.g. through a nozzle or one or more membranes of appropriate pore size, in order to enhance the uniformity of the size distribution of the microparticles and/or microballoons which are ultimately obtained.

The contrast agents of the invention may be stored and transported in dry form, in which condition they will normally be stable for long periods, being mixed with an appropriate liquid carrier (e.g. sterile water for injection, physiological saline or phosphate buffer) prior to administration. In this way the concentration of the injected or otherwise administered contrast agent may be varied at will, depending on the precise nature of the application. They may also be stored in suspension in such carriers, being substantially completely stable in aqueous media in the absence of esterase enzymes.

Polymers consisting of repeating units of formula (II) as hereinbefore defined are themselves novel products and constitute a further feature of the invention. As well as being useful starting materials for preparing contrast agents according to the invention, such polymers may be of use as or in, for example, surgical implants such as sutures, soft tissue prostheses, sponges, films (e.g. artificial skin), wound dressings (e.g. hydrogel sheets), flexible sheet materials and articles such as containers formed therefrom, delayed release formulations for drugs and agricultural chemicals, particulate imaging agents or plasticisers.

The novel polymers may be prepared by any convenient method, for example by reaction of a reactive derivative such as a dihalide of a diacid of formula

HOOC.(CH₂)_{b}.COOH

(where b is as hereinbefore defined) with a diol of formula

HO. (CH₂)ₐ.CO.O.CH(CH₃) .O.CO. (CH₂)ₐ.OH

(where a is as hereinbefore defined), e.g. in an appropriate organic solvent. The diol may itself be prepared by reacting an ethylidene halide such as the iodide with two moles of ω-hydroxyacid HO.(CH₂)ₐ.COOH, e.g. in the presence of a base.

The following non-limitative Examples serve to illustrate the invention.

### EXAMPLE 1 - Preparation of intermediates

### a) Ethylidene bis (16-hydroxyhexadecanoate)

1,8-Diazabicyclo [5.4.0]undec-7-ene (1,5-5) (DBU) (2.74 g, 0.018 mol) was added to 16-hydroxyhexadecanoic acid (4.90 g, 0.018 mol) in dimethylformamide (150 ml). After 5 minutes with stirring, ethylidene iodide (2.54 g, 0.009 mol) was added and the mixture was left with stirring at 40°C for 3 days. The reaction mixture was cooled to 20°C and when precipitation was complete (2 hours) the precipitated monomer was isolated by filtration. The monomer was treated with activated carbon and recrystallised twice from dichloromethane to give 1.03 g (20%) of the title product. Differential scanning calorimetry (DSC) indicated that onset melting temperature was 88.93°C. ¹H NMR (200 MHz, CDCl₃) : δ 1.25 (s, 44H, CH₂), 1.45 (d, 3H, CH₃CH), 1.56 (m, 8H, CH₂), 2.30 (t, 4H, CH₂CO), 3.63 (t, 4H, 2 X CH₂O), 6.86 (q, 1H, CHCH₃). ¹³C NMR (50 MHz, CDCl₃): δ 20.86, 25.91, 26.98, 30.22, 30.44, 30.67, 30.84, 34.00, 35.30, 64.00, 89.00, 171.77 (C=O).

### b) Ethylidene bis [16-(5-chlorocarbonylpentanoyloxy)-hexadecanoate]

In a three-necked round bottomed flask equipped with a reflux condenser, a glass gas inlet tube and a pressure equalizing dropping funnel was placed freshly distilled adipoyl chloride (2.60 ml, 17.50 mmol) dissolved in absolute chloroform (15 ml). The temperature was raised to ca. 50°C and under a gentle stream of nitrogen through the solution, a solution of ethylidene bis(16-hydroxyhexadecanoate) (1.0g, 1.75 mmol) in absolute chloroform (30 ml) was added dropwise and left at this temperature a further 3 hours after addition. The mixture was then cooled to room temperature and quickly transferred into a 50 ml round bottomed flask equipped for distillation under reduced pressure. Chloroform was first distilled off at normal pressure, then oil-pump vacuum was established and excess adipoyl chloride distilled off at ca. 75°C, 5 mbar pressure, leaving the residual title compound (1.56g).

### c) 16-Hexadecanoyloxyhexadecanoic acid

16-Hydroxyhexadecanoic acid (5.43g, 19.9 mmol) was dissolved in tetrahydrofuran (190 ml) and pyridine (2.36g, 29.9 mmol) was added. Palmitoyl chloride (5.48g, 19.9 mmol) was dissolved in tetrahydrofuran (10 ml) and added dropwise at room temperature. After stirring at room temperature for 16 hours, the mixture was filtered and the filtrate evaporated under reduced pressure. The residue was dissolved in chloroform, washed with water (3 x 50 ml), and the organic phase was dried (MgSO₄). After evaporating under reduced pressure, the residue was purified on a silica column, eluting with chloroform with increasing methanol concentration (from 1% to 2% methanol in chloroform) to give 8.41g (83%) of the title compound. ¹H NMR (300 MHz, CDCl₃) : δ 0.85 (t, 3H, CH₃), 1.20-1.35 (s, 46H, -CH₂-), 1.55-1.70 (m, 6H, -CH₂-), 2.25 (t, 2H, -CH₂-C(O)-O), 2.45 (t, 2H, -CH₂-COOH), 4.05 (t, 2H, -O-CH₂). ¹³C NMR (75 MHz, CDCl₃): δ 14.01, 22.57, 24.10, 24.91, 25.82, 28.53, 28.75, 28.94, 29.08, 29.15, 29.25, 29.36, 29.54, 31.81, 34.29, 35.16, 64.27, 76.48, 76.90, 77.10, 77.32, 169.50, 173.91.

### d) 16-Hexadecanoyloxyhexadecanoyl chloride

16-Hexadecanoyloxyhexadecanoic acid (7.73g, 15.13 mmol) prepared as in (c) above was dissolved in tetrahydrofuran (140 ml) and oxalyl chloride (4.80g, 37.83 mmol) was added dropwise. The mixture was stirred at room temperature for 3 days and then the solvent and unreacted oxalyl chloride were evaporated under reduced pressure to give 8.0g (100%) of the title compound.

### e) 1-[16-(16-Hexadecanoyloxyhexadecanoyloxy)-hexadecanoyloxy]ethyl 16-hydroxyhexadecanoate

Ethylidene bis(16-hydroxyhexadecanoate) (4.38g, 7.67 mmol) was dissolved in tetrahydrofuran (80 ml) and pyridine (0.61g, 7.71 mmol) was added. 16-Hexadecanoyloxyhexadecanoyl chloride (4.18g, 7.90 mmol) was dissolved in tetrahydrofuran (20 ml) and added dropwise. After 3 days at room temperature the mixture was filtered and the filtrate was left at -20°C for 2 hours. The precipitated product was filtered and purified by flash chromatography (silicagel, chloroform) to give 2.4g (29%) of the title compound. ¹H NMR (300 MHz, CDCl₃) : δ 0.85 (t, 3H, CH₃), 1.2-1.4 (s, 90H, -CH₂-), 1.45 (d, 3H, -O-CH(CH₃)-O-), 1.5-1.7 (m, 14H, -CH₂-), 2.25 (m, 8H, -CH₂-C(O)-O-), 3.60 (t, 2H, -CH₂-OH), 4.05 (t, 4H, -C(O)-O-CH₂-), 6.85 (q, 1H, -O-CH(CH₃)-O-). ¹³C NMR (75 MHz, CDCl₃) : δ 13.7, 19.1, 22.2, 24.2, 24.6, 25.2, 25.5, 28.2, 28.5, 28.7, 28.8, 29.0, 29.2, 31.5, 32.3, 33.7, 34.0, 62.5, 64.0, 88.0, 171.5, 173.5.

### f) Preparation of Methoxy-endcapped polyethylene glycols (PEGs)

### Preparation of a Tyical Polymer (MeO-PEG 2000)

An initiator solution was prepared by careful addition of potassium metal (0.400 g, 10.23 mmol) to methanol (1.300g, 40.57 mmol) in an inert atmosphere. A portion of this initiator solution (0.220g, 1.32 mmol potassium methoxide) was injected into an ampoule containing ethylene oxide (10.000g, 227.00 mmol). The sealed ampoule was allowed to stand at room temperature overnight. The temperature was then raised to 60°C and reaction allowed for 72 hours. After removal of unreacted monomer, the contents of the ampoule were dissolved in dichloromethane and the solution neutralised with dilute aqueous hydrochloric acid. The polymer solution was washed three times with distilled water, rotary evaporated and then vacuum dried. Assignments for MeO-PEG polymers. ¹H-NMR: δ 2.7 (OH), 3.2 (OCH₃), 3.5 (-CH₂- main chain), 3.4 (-CH₂OCH₃). ¹³NMR: δ 58.5 (-OCH₃), 61.2 (-CH₂OH), 70.5 (-CH₂- main chain), 71.3 (-CH₂OCH₃), 72.2 (-CH₂CH₂OH). The GPC was recorded in THF and the molecular weight calibration was via PEG standards. GPC data for a typical sample: Mp: 2679, Mn: 2012, Mw: 2283. Polydispersity: 1.135.

### g) General procedure for methoxy PEG chloroformate

PEG 2000 monomethyl ether (6.00g, 3.00 mmol) was dissolved in toluene (50 ml) and dried by refluxing in a Dean Stark apparatus. Pyridine (0.24g, 3.00 mmol) was added at room temperature. Trichloromethyl chloroformate ("diphosgene") (0.60g, 3.00 mmol) was dissolved in toluene (10 ml) and added dropwise. The mixture was stirred at room temperature for 12 hours and filtered. The solvent was evaporated under reduced pressure to give the title compound in quantitative yield.

### EXAMPLE 2 - Preparation of polymers and emulsifiers

### a) Polymer from ethylidene bis(16-hydroxyhexadecanoate) and adipoyl chloride

A solution of adipoyl chloride (0.48 g, 2.6 mmol) in xylene/trichloroethylene (80:20 v/v, 5ml) was added to a solution of ethylidene bis(16-hydroxyhexadecanoate) (1.48 g, 2.6 mmol) from Example 1(a) above in xylene/ trichloroethylene (80:20 v/v, 100 ml) at 60°C. After 2 days at 60°C under reduced pressure (147 mbar), the reaction mixture was cooled to 20°C. The solvent was evaporated under reduced pressure, the resulting polymer was dissolved in chloroform, reprecipitated in hexane and filtered, giving 1.05 g (60%) of the title compound as a white powder. Size Exclusion Chromatography (SEC): Mw=39068, Mn=9442, Mp=48536, Mw/Mn=4.138 (using polystyrene as standards). Differential scanning calorimetry (DSC) indicated that onset melting temperature was 48.61°C. ¹H NMR (200 MHz, CDCl₃) : δ 1.28 (s, 44H, CH₂), 1.45 (d, 3H, CH₃CH), 1.62 (m, 12H, CH₂), 2.32 (m, 8H, CH₂CO), 4.02 (t, 4H,2 X CH₂O), 6.88 (q, 1H, CHCH₃). ¹³C NMR (50MHz, CDCl₃) : δ 20.85, 25.64, 25.68; 25.89, 27.16, 29.84, 30.15, 30.21, 30.44, 30.81, 35.08, 35.12, 35.27, 65.45, 88.98, 171.77 (C=O), 173.41 (C=O).

### b) Random chain-extended polymer of PEG 1500, adipoyl chloride and ethylidene bis (16-hydroxyhexadecanoate) (0.37:1.85:1.75), multiblock

To a suspension of ethylidene bis(16-hydroxyhexadecanoate) (1.0g, 1.75 mmol) in dimethoxyethane (10 ml) at room temperature was added freshly distilled adipoyl chloride (270 µl, 1.85 mmol). The temperature of the mixture was gradually raised to 60°C, and a colourless solution obtained. After 5 hours at this temperature PEG 1500 (0.55g, 0.37 mmol) was added and heating continued for a further 17 hours before the mixture was cooled to room temperature, the solvent evaporated and the solid residue stirred in petroleum ether (bp 40-60°C) for 15 minutes and filtered to give the title compound (1.30g) as a white solid.

### c) Extended polymer from PEG 1500 and ethylidene bis [16-(5-chlorocarbonylpentanoyloxy)-hexadecanoatel (A-B-A)

Ethylidene bis[16-(5-chlorocarbonylpentanoyloxy)-hexadecanoate] prepared as in Example 1(b) (0.88 g, 1.02 mmol) was dissolved in toluene (15 ml) in a 100 ml 3-necked round bottomed flask equipped with a glass gas inlet tube and a reflux condenser. PEG 1500 (3.06g, 2.04 mmol) was added and the mixture heated at 60°C for 22 hours, cooled to room temperature and the solvent removed under reduced pressure to give the title compound (4.12g) as a white wax.

### d) Extended polymer from PEG 1500 and ethylidene bis [16-(5-chlorocarbonylpentanoyloxy)hexadecanoatel (multiblock)

The reaction was performed as in Example 2(c), but with ethylidene bis[16-(5-chlorocarbonylpentanoyloxy)-hexadecanoate (1.02g, 1.18 mmol) in toluene (20 ml) and PEG 1500 (1.77g, 1.18 mmol) to give the title compound (2.29g) as a white wax.

### e-h) Extended polymer of PEG, adipic acid and ethylidene bis (16-hydroxyhexadecanoate) (random multiblock)

A solution of PEG of appropriate molecular weight (A) (2.07 mmol) in the stated solvent (26 ml) was added via a syringe to a round bottomed flask containing ethylidene bis(16-hydroxyhexadecanoate) (B) (118 mg, 0.207 mmol), under nitrogen atmosphere. The resulting mixture was heated to 60°C, and when a clear solution had been obtained, adipoyl chloride (C) (417 mg, 2.277 mmol) was added via a syringe. The pressure was reduced to 250 mbar and the solution was stirred at 60°C for the stated period. Remaining hydrogen chloride, evolved in the reaction, and the solvent were removed on a rotatory evaporator at reduced pressure and 60°C for 3 hours, and subsequently under vacuum (<0.1mm Hg) at 60°C for 24 hours. Finally, the polymer was precipitated from an acetone solution by adding petroleum ether, and cooling in an ice bath for 2 hours. Filtration yielded 3.5g of the polymer as a white waxy solid.

In total four different block copolymers differing in the molecular weight of the starting PEGs were prepared by this method; the conditions specific for each polymerisation are given in Table 1 below. ¹³C NMR- and ¹H NMR-spectra of the polymers was in agreement with the expected products.

**Table 1**

| Entry | Mw for starting PEG | Molar ratio A:B:C¹ | Solvent | Reaction time (hours) |
|---|---|---|---|---|
| e | 400 | 10:1:11 | Diglyme-xylene | 21 |
| f | 600 | 10:1:11 | Diglyme | 24 |
| g | 1500 | 10:1:11 | Dimethoxyethane | 21 |
| h | 2000 | 10:1:11 | 1,1,2-Trichloroethylene | 92 |

| | | | | |
|---|---|---|---|---|
| ¹) The letters refers to the reactants as specifed in the text above. | | | | |

### i) PEG 2300 methyl ether 16-hexadecanoyloxyhexadecanoate

PEG 2300 methyl ether (10.000g, 4.35 mmol) was dissolved in tetrahydrofuran (90 ml) and pyridine (0.413g, 5.22 mmol) was added. 16-Hexadecanoyloxyhexadecanoyl chloride (2.301g, 4.35 mmol) was dissolved in tetrahydrofuran (10 ml) and added dropwise. After stirring for 3 days at room temperature, the mixture was filtered and the solvent was evaporated under reduced pressure. The residue (12.08g) was purified on a silica column, eluting with chloroform with increasing methanol concentration (from 1% to 3% methanol in chloroform) to give 5.20g (43%) of the title compound. ¹H NMR (300 MHz, CDCl₃) : δ 0.80-0.87 (m, CH₃), 1.21 (s, (br), CH₂), 1.53-1.62 (m, CH₂), 2.20-2.35 (m, CH₂CO), 3.34 (s, CH₃O), 3.61 (s, OCH₂CH₂O), 4.02 (t, COOCH₂CH₂O), 4.19 (t, COOCH₂CH₂O). ¹³C NMR (75 MHz, CDCl₃) : δ 13.95, 22.49, 24.71, 24.83, 25.74, 28.45, 28.95, 29.07, 29.16, 29.28, 29.34, 29.40, 29.46, 31.72, 34.05, 34.21, 58.85, 63.15, 64.19, 69.01, 70.37, 71.73, 173.64, 173.82.

### j) PEG 5000 methyl ether 16-hexadecanoyloxyhexadecanoate)

PEG 5000 methyl ether (7.500g, 1.50 mmol) was dissolved in toluene (90 ml) and dried by refluxing in a Dean Stark apparatus. Pyridine (0.143g, 1.80 mmol) was added followed by addition (dropwise) of 16-hexadecanoyloxyhexadecanoyl chloride (1,191g, 2.25 mmol) dissolved in toluene (10 ml). The mixture was heated to reflux and after stirring under reflux for 3 days the mixture was cooled to room temperature and precipitated into hexane. After filtering, the precipitate was washed with hexane and dried (MgSO₄). After evaporation under reduced pressure, the residue was purified on a silica column, eluting with chloroform with increasing methanol concentration (from 1% to 3% methanol in chloroform) to give 5.93g (72%) of the title compound. ¹H NMR (300 MHz, CDCl₃) : δ 0.82-0.86 (m, CH₃), 1.22 (s, (br), CH₂), 1.53-1.62 (m, CH₂), 2.20-2.35 (m, CH₂CO), 3.34 (s, CH₃O), 3.61 (s, OCH₂CH₂O), 4.01 (t, COOCH₂CH₂O), 4.18 (t, COOCH₂O). ¹³C NMR (75 MHz, CDCl₃) : δ 13.66, 22.21, 24.43, 24.54, 25.46, 28.17, 28.67, 28.79, 28.87, 28.99, 29.06, 29.11, 29.17, 31.44, 33.73, 33.93, 58.57, 62.87, 63.90, 68.72, 69.62, 69.86, 70.09, 71.45, 76.85, 173.35, 173.53.

### k) PEG 10000 methyl ether 16-hexadecanoyloxyhexadecanoate

PEG 10000 methyl ether (7.500g, 0.75 mmol) was dissolved in toluene (140 ml) and pyridine (0.107g, 1.35 mmol) was added. The solution was heated to 60°C and 16-hexadecanoyloxyhexadecanoyl chloride (0.595g, 1.12 mmol) dissolved in toluene (10 ml) was added dropwise. The mixture was heated to reflux and after stirring under reflux for 3 days the mixture was cooled to room temperature and precipitated into hexane. After filtering, the precipitate was washed with hexane and dried. Flash chromatography on a silica column, eluting with 5% methanol in chloroform, gave 5.39g (68%) of the title compound. ¹H NMR (300 MHz, CDCl₃) : δ 0.84 (t, CH₃), 1.21 (s, (br), CH₂), 1.55-1.60 (m, CH₂), 2.20-2.35 (m, CH₂CO), 3.34 (s, CH₃O), 3.61(s, OCH₂CH₂O), 4.01 (t, COOCH₂CH₂O), 4.18 (t, COOCH₂CH₂O). ¹³C NMR (75 MHz, CDCl₃) : δ 13.94, 22.48, 24.70, 24.82, 25.73, 28.94, 29.05, 29.14, 29.26, 29.33, 29.39, 29.45, 31.71, 34.00, 58.84, 63.14, 68.99, 69.36, 69.86, 69.97, 70.01, 70.36, 70.74, 70.82, 70.86, 71.72, 77.10, 173.62, 173.80.

### l) 16-[ω-Methoxy-PEG 2000-carbonyloxy]hexadecanoic acid 1-[16-(16-hexadecanoyloxyhexadecanoyloxy)-hexadecanoyloxylethyl ester

Methoxy PEG 2000 chloroformate (1.90g, 0.95 mmol) was dissolved in toluene (90 ml), and pyridine (0.09g, 1.13 mmol) was added. 1[[16-(16-Hexadecanoyloxyhexadecanoyloxy)hexadecanoyloxy]ethyl 16-hydroxyhexadecanoate (1.00g, 0.95 mmol) was dissolved in toluene (10 ml) and added dropwise. The mixture was heated to reflux and after stirring under reflux for 10 hours, the mixture was cooled to room temperature and filtered. The solvent was evaporated under reduced pressure. The residue was purified on a silica column using chloroform containing 2% methanol, to give 1.00g (35%) of the title compound. ¹H-NMR (300 MHz, CDCl₃) : δ 0.85 (t, CH₃), 1.20-1.33 (m, CH₂), 1.45 (d, -O-CH(CH₃)-O), 1.5-1.7 (m, CH₂), 2.0 (H₂O), 2.2-2.3 (m, -CH₂-C(O)-O), 3.35 (s, CH₃-O-), 3.5-3.7 (s, -OCH₂CH₂O-), 4.03 (t, -C(O)-O-CH₂-), 4.10 (t, -CH₂-O-C(O)-O-), 4.26 (m, -O-C(O)-O-CH₂-CH₂-O-), 6.8-6.9 (q, -O-CH(CH₃)-O). ¹³C-NMR (75 MHz, CDCl₃): δ 13.7, 19.2, 22.1, 24.2, 24.6, 25.2, 25.5, 28.2-29.2, 31.5, 33.9, 34.0, 58.7, 64.0, 66.3, 67.9, 68.5, 70.0, 71.5, 87.9, 171.5, 173.7.

### m) 16-[ω-Methoxy PEG 5000 carbonyloxy]hexadecanoic acid 1-[16-(16-hexadecanoyloxyhexadecanoyloxy)-hexadecanoyloxy]ethyl ester

Methoxy PEG 5000 chloroformate (8.50g, 1.70 mmol) was dissolved in toluene (90 ml) and pyridine (0.146g, 1.85 mmol) was added. 1-[16-(16-Hexadecanoyloxyhexadecanoyloxy)hexadecanoyloxy]ethyl 16-hydroxyhexadecanoate (1.79g, 1.70 mml) was dissolved in toluene (10 ml) and added dropwise. The mixture was heated to reflux and after stirring under reflux for 3 days the mixture was cooled to room temperature and filtered. The solvent was evaporated under reduced pressure and the residue was purified on a silica column, eluting with chloroform with increasing methanol concentration (from 3% to 5% methanol in chloroform) to give 3.90g (38%) of the title compound. ¹H-NMR (300 MHz, CDCl₃) : δ 0.85 (t, CH₃), 1.20-1.33 (m, CH₂), 1.45 (d, -O-CH(CH₃)-O), 1.5-1.7 (m, CH₂), 1.8 (H₂O), 2.2-2.3 (m, -CH₂-C(O)-O), 3.35 (s, CH₃-O-), 3.5-3.7 (s, -OCH₂CH₂O-), 4.03 (t, -C(O)-O-CH₂-), 4.10 (t, -CH₂-O-C(O)-O-), 4.26 (m, -O-C(O)-O-CH₂-CH₂-O-), 6.8-6.9 (q, -O- CH(CH₃)-O).

### n) 16-[ω-Methoxy PEG 10000 carbonyloxy]hexadecanoic acid 1-[16-(16-hexadecanoyloxyhexadecanoyloxy) hexadecanoyloxylethyl ester

Methoxy PEG 10000 chloroformate (7.50g, 0.75 mmol) was dissolved in toluene (90 ml), and pyridine (0.063g, 0.80 mmol) was added. 1-[16-(16-Hexadecanoyloxyhexadecanoyloxy)hexadecanoyloxy]ethyl 16-hydroxyhexadecanoate (0.79g, 0.75 mmol) was dissolved in toluene (10 ml) and added dropwise. The mixture was heated to reflux and after stirring under reflux for 3 days the mixture was cooled to room temperature and filtered. The solvent was evaporated off under reduced pressure. The residue was purified on a silica column, eluting with chloroform with increasing methanol concentration (from 3% to 5% methanol in chloroform) to give 1.60g (19%) of the title compound. ¹H-NMR (300 MHz, CDCl₃) : δ 0.85 (t, CH₃), 1.20-1.33 (m, CH₂), 1.45 (d, -O-CH(CH₃)-O), 1.5-1.7 (m, CH₂), 2.2-2.3 (m, -CH₂-C(O)-O), 3.35 (s, CH₃-O-), 3.5-3.7 (s, -OCH₂CH₂O-), 4.03 (t, -C(O)-O-CH₂), 4.10 (t, -CH₂-O-C(O)-O-), 4.26 (m, -O-C(O)-O-CH₂-CH₂-O-), 6.8-6.9 (q, -O-CH(CH₃)-O).

### EXAMPLE 3 - Preparation of polymer particles

### a) Particles from polymer made from ethylidene bis (16-hydroxyhexadecanoate) and adipoyl chloride

10 ml of a 5% w/v solution of the polymer from Example 2(a) in toluene was added to 30 ml of a 5 wt% solution of human serum albumin in water. The two phases were mixed with an Ultra Turax® T25 mixer at 20,000 rpm for 1 minute, frozen on a dry ice/methanol bath, and lyophilized for 18 hours, giving a slightly yellow powder. Light microscopy indicated formation of microparticles.

### b) Particles from polymer made from ethylidene bis (16-hydroxyhexadecanoate) and adipoyl chloride

10 ml of a 10% w/v solution of the polymer from Example 2(a) in p-xylene was added to 30 ml of a 5 wt% solution of human serum albumin in water. The mixture was mixed with an Ultra Turax® T25 mixer at 20,000 rpm for 1 minute and 30 seconds, frozen on a dry ice/methanol bath, and lyophilized for 18 hours, giving a white powder. Light microscopy indicated formation of microparticles.

### c) Particles from polymer made from ethylidene bis (16-hydroxyhexadecanoate) and adipoyl chloride

10 ml of a 5% w/v solution of the polymer from Example 2(a) in p-xylene was added to 30 ml of a 5 wt% solution of modified starch (Lyckeby, Sweden, PU-24.000) in water. The mixture was mixed with an Ultra Turax® T25 mixer at 20,000 rpm for 1 minute and 30 seconds, frozen on a dry ice/methanol bath, and lyophilized for 18 hours, giving a white powder. Light microscopy indicated formation of microparticles.

### d) Particles from polymer made from ethylidene bis (16-hydroxyhexadecanoate) and adipoyl chloride

10 ml of a 5% w/v solution of the polymer from Example 2(a) in p-xylene was added to 30 ml of a 0.8 wt% solution of polyvinyl alcohol in water. The mixture was mixed with an Ultra Turax® T25 mixer at 20,000 rpm for 1 minute, frozen on a dry ice/ methanol bath, and lyophilized for 18 hours, giving a white powder. Light microscopy indicated formation of microparticles.

### e) Particles from polymer made from ethylidene bis (16-hydroxyhexadecanoate) and adipoyl chloride

10 ml of a 5% w/v solution of the polymer from Example 2(a) in p-xylene was added to 30 ml of a 1 wt% solution of gelatin in water. The mixture was mixed with an Ultra Turax® T25 mixer at 20,000 rpm for 1 minute, frozen on a dry ice/methanol bath, and lyophilized for 18 hours, giving a white powder. Light microscopy indicated formation of microparticles.

### f) Particles from polymer made from ethylidene bis (16-hydroxyhexadecanoate) and adipoyl chloride

5 ml of a 5% w/v solution of the polymer from Example 2(a) in (-)-camphene maintained at 60°C was added to 15 ml of a 5 wt% solution of human serum albumin in water at the same temperature. The mixture was mixed hot with an Ultra Turax® T25 mixer at 20,000 rpm for 1 minute, frozen on a dry ice/methanol bath, and lyophilized for 48 hours, giving a white powder. Light microscopy indicated formation of microparticles.

### g-n) Particles from polymer made from ethylidene bis (16-hydroxyhexadecanoate) and adipoyl chloride, stabilized in dispersion with block copolymer

### General description

10 ml of a 5% w/v solution of the polymer from Example 2(a) in (-)-camphene maintained at 60°C was added to 30 ml of an aqueous solution of block copolymer from Example 2 above (see Table 2) at the same temperature and with concentrations as given in Table 2. The mixture was mixed with a rotor-stator mixer (Ultra Turax® T25) at slow speed for several minutes, frozen on a dry ice/ methanol bath, and lyophilized for 48 hours, giving a white powder.

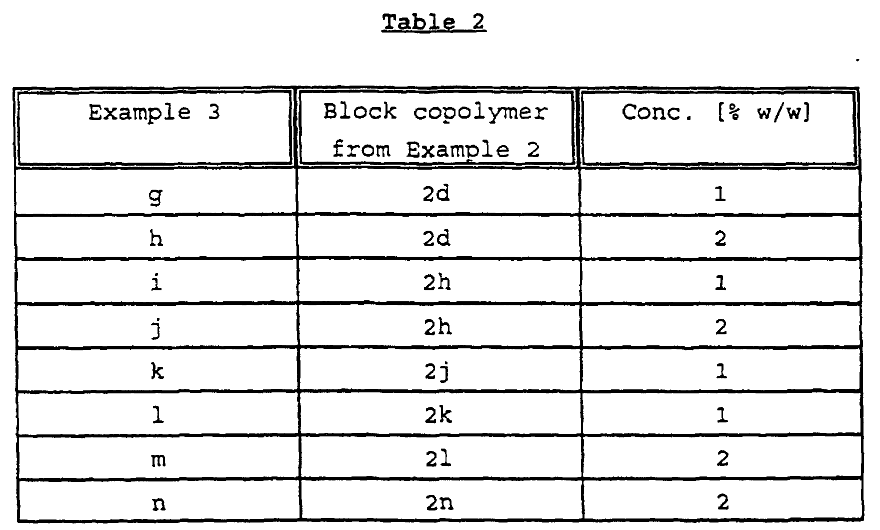

### o) Particles from polymer made from ethylidene bis (16-hydroxyhexadecanoate) and adipoyl chloride

16ml of a 3% w/v solution of the polymer from Example 2(a) in (-)-camphene maintained at 70°C was added to 64 ml of an aqueous solution containing 1% w/v of the block copolymer from Example 2(k) and 5% w/v of PEG 3000 at the same temperature. The mixture was mixed with a rotor-stator mixer at moderate speed for up to 5 minutes, frozen on a dry ice/methanol bath, and lyophilized for 48 hours, giving a white powder. The dry product was dispersed in saline solution on a laboratory shaker for 16 hours at a concentration of 10 mg dry material/ml.

### p) Particles from polymer made from ethylidene bis (16-hydroxyhexadecanoate) and adipoyl chloride

The procedure of Example 3 (o) was repeated, but with cyclooctane in place of (-)-camphene as organic solvent.

### q) Particles from polymer made from ethylidene bis (16-hydroxyhexadecanoate) and adipoyl chloride

The procedure of Example 3(o) was repeated, but with cyclohexane in place of (-)-camphene as organic solvent.

### r) Particles from polymer made from ethylidene bis (16-hydroxyhexadecanoate) and adipoyl chloride

The procedure of Example 3(o) was repeated, except that emulsification was carried out at 60°C using 28 ml of a 7.5% w/v solution of the polymer from Example 2(a) in (-)-camphene and 62 ml of an aqueous solution containing 2% w/v of the polymer from Example 2(k).

### EXAMPLE 4 - Acoustic characterizations.

### General procedure

Dry powders of polymer particles prepared according to Example 3 above were redispersed to 10 mg/ml dry material in MilliQ water by shaking on a laboratory shaker for 12-16 hours. Examination by light microscopy indicated formation of particle dispersions. The particles floated readily, as expected for gas-containing particles.

### Acoustic effects in vitro.

The acoustic effect of suspensions prepared as above was obtained by measuring the ultrasonic transmission through solutions of different concentrations (mg/ml) in an aqueous carrier liquid, using a 3.5 MHz broadband transducer in a pulse-reflection technique. The aqueous carrier liquid was used as reference, and measurements were performed on serial dilutions with the carrier liquid until the signal was reduced to approximiately 3-5 db/cm. The concentration necessary to give an attenuation of 8 db/cm was noted (Table 3); hence low values indicate a good contrast effect. The obtained acoustic effects are at a level indicating that the products can be expected to be useful as ultrasound contrast agents. According to theoretical considerations, solid (as opposite to gas-containing) particles of the same size and at the same dilutions should give an acoustic attenuation of less than 0.1 db/cm.

### Table 3

Acoustic measurements of particles from Example 3 above. The acoustic measurements are given in column 3 as the concentration giving a contrast effect of 8 db/cm, i.e half value of saturated signal. At higher concentrations, the signal intensity increased until saturation was observed.

| **Example 4** | **Particles of Example** | **Particle conc. at 8 db/cm [mg/ml]** |
|---|---|---|
| a | 3a | 1.0 |
| b | 3b | 0.12 |
| c | 3c | 0.03 |
| d | 3d | 0.16 |
| e | 3e | 0.35 |
| f | 3f | 0.15 |
| g | 3g | 0.04 |
| h | 3h | 0.02 |
| i | 3i | 0.02 |
| j | 3j | 0.02 |
| k | 3k | 0.03 |
| l | 3l | 0.01 |
| m | 3m | 0.09 |
| n | 3n | 0.08 |

### EXAMPLE 5 - In vivo characterizations

The powders of polymer particles prepared as described in Example 3(a)-(f) above were redispersed in sterile 0.9% (w/v) NaCl (aq) solution by shaking on a laboratory shaker for 12-16 hours. The dispersions were injected in leg veins of dogs, and short axis transthoracic echocardiac images were obtained using a Vingmed Sound CFM750 ultrasonic scanner at 5 MHz. The image sequences were stored on video. The particle dispersions all caused very sharp contrast enhancement in both ventricles and also caused significant myocardial contrast enhancement (MCE), visible on live video sequences. MCE was demonstrated in the anterior and posterior walls. The duration of contrast effect reveals that the particle dispersons circulated in vivo for several minutes after injection. The presence of myocardial contrast and the long duration of contrast indicates that the in vivo stability is very good.

## Claims

1. A contrast agent comprising gas-containing polymer microparticles and/or microballoons **characterised in that** the polymer is a biodegradable polymer consisting of repeating units of formula (II) 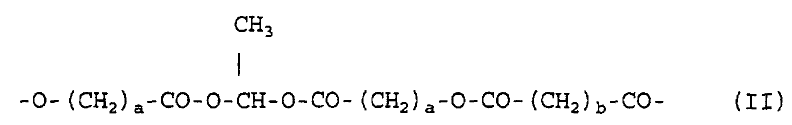 (where a represents an integer in the range 9-19 and b represents an integer in the range 1-8).

2. A contrast agent as claimed in claim 1 wherein a represents an integer in the range 13-17 and b represents an integer in the range 3-6.

3. A contrast agent as claimed in claim 1 wherein a is 15 and b is 4.

4. A contrast agent as claimed in any of claims 1 to 3 incorporating one or more additives selected from emulsifying agents, coating agents, plasticisers, bulking agents, cryoprotectants and antioxidants.

5. A contrast agent as claimed in claim 4 incorporating an emulsifying agent selected from fatty acids, carbohydrate esters of fatty acids, triglyceride esters of fatty acids, proteins, phospholipids, polysaccharides and surface active polymers.

6. A contrast agent as claimed in claim 5 wherein the emulsifying agent is human serum albumin, modified starch or gelatin.

7. A contrast agent as claimed in claim 5 wherein the emulsifying agent is polyvinyl alcohol or a block copolymer/extended polymer.

8. A contrast agent as claimed in claim 7 wherein the block copolymer/extended polymer contains polyethylene glycol units as the hydrophilic blocks and units of formula (III)
-R^{a}-(CH₂)ₐ-CO-O-CH(CH₃)-O-CO-(CH₂)ₐ-R^{b}- (III)
(where a is as defined in claim 1 and R^{a} and R^{b} each represent valence bonds or linking groups) in the hydrophobic part as an extending moiety or as a moiety in an oligomeric or polymeric block.

9. A contrast agent as claimed in claim 8 wherein R^{a} and R^{b} are each selected from carbonyl groups and groups of formula -O-CO-(CH₂)_{b}-CO-O- (where b is as defined in claim 1).

10. A contrast agent as claimed in claim 7 wherein the emulsifying agent is an extended polymer comprising a methoxy-terminated polyethylene glycol hydrophilic block acylated with a hydrophobic moiety comprising a chain of two or more fatty acids.

11. A contrast agent as claimed in claim 10 wherein the hydrophobic moiety is an acyloxyacyl group.

12. A contrast agent as claimed in claim 11 wherein the hydrophobic moiety is 16-hexadecanoyloxyhexadecanoyl.

13. A process for the preparation of a contrast agent as claimed in claim 1 which comprises incorporation of a gas into a biodegradable polymer comprising repeating units of formula (II) as defined in claim 1 so as to form polymer microparticles and/or microballoons.

14. A process as claimed in claim 13 which comprises emulsifying a solution of the polymer in a water-immiscible organic solvent in an aqueous phase and thereafter removing at least the organic phase under an atmosphere of the gas which is to be incorporated.

15. A process as claimed in claim 14 wherein the aqueous phase contains an emulsifying agent as defined in any of claims 5 to 12.

16. A process as defined in claim 14 or claim 15 wherein the emulsion is filtered and/or extruded prior to phase removal.

17. Biodegradable polymers consisting of repeating units of formula (II) as defined in claim 1.

18. Biodegradable polymers as claimed in claim 17 wherein a represents an integer in the range 13-17 and b represents an integer in the range 3-6.

19. Biodegradable polymers as claimed in claim 17 wherein a is 15 and b is 4.

20. A surgical implant, soft tissue prosthesis, sponge, film, wound dressing, flexible sheet, container, medical or agricultural delayed release formulation, particulate imaging agent or plasticiser comprising a polymer as claimed in any of claims 17 to 19.

21. A process for the preparation of a polymer as claimed in claim 17 which comprises reacting a reactive derivative of a diacid of formula
HOOC.(CH₂)_{b}.COOH
(where b is as defined in claim 1) with a diol of formula
HO.(CH₂)ₐ.CO.O.CH(CH₃).O.CO.(CH₂)ₐ.OH
(where a is as defined in claim 1).

## Patentansprüche

1. Kontrastmittel, umfassend gashaltige Polymermikroteilchen und/oder Mikroballoons, **dadurch gekennzeichnet, daß** das Polymer ein bioabbaubares Polymer ist, bestehend aus wiederkehrenden Einheiten der Formel (II) 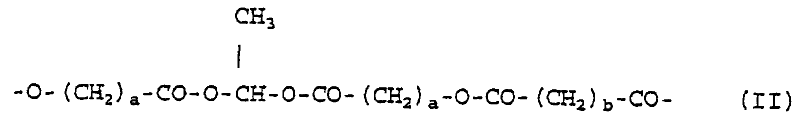 (worin a eine ganze Zahl im Bereich von 9-19 bedeutet und b eine ganze Zahl im Bereich von 1-8 bedeutet).

2. Kontrastmittel nach Anspruch 1, wobei a eine ganze Zahl im Bereich von 13-17 bedeutet und b eine ganze Zahl im Bereich von 3-6 bedeutet.

3. Kontrastmittel nach Anspruch 1, wobei a 15 ist und b 4 ist.

4. Kontrastmittel nach mindestens einem der Ansprüche 1-3, beinhaltend ein oder mehrere Additive, gewählt aus Emulgiermitteln, Beschichtungsmitteln, Weichmachern, Füllmitteln, Kryoschutzmitteln und Antioxidantien.

5. Kontrastmittel nach Anspruch 4, beinhaltend ein Emulgiermittel, gewählt aus Fettsäuren, Kohlenhydratestern von Fettsäuren, Triglyceridestern von Fettsäuren, Proteinen, Phospholipiden, Polysacchariden und oberflächenaktiven Polymeren.

6. Kontrastmittel nach Anspruch 5, wobei das Emulgiermittel Humanserumalbumin, modifizierte Stärke oder Gelatine ist.

7. Kontrastmittel nach Anspruch 5, wobei das Emulgiermittel Polyvinylalkohol oder ein Blockcopolymer/Extended Polymer ist.

8. Kontrastmittel nach Anspruch 7, wobei das Blockcopolymer/Extended Polymer Polyethylenglykoleinheiten als die hydrophilen Blöcke und Einheiten der Formel (III)
-R^{a}-(CH₂)ₐ-CO-O-CH(CH₃)-O-CO-(CH₂)ₐ-R^{b}- (III)
(worin a wie in Anspruch 1 definiert ist, und R^{a} und R^{b} jeweils Valenzbindungen oder Verbindungsgruppen bedeuten) in dem hydrophoben Teil als eine Extensionseinheit oder als eine Einheit in einem oligomeren oder polymeren Block enthält.

9. Kontrastmittel nach Anspruch 8, wobei R^{a} und R^{b} jeweils gewählt sind aus Carbonylgruppen und Gruppen der Formel -O-CO-(CH₂)_{b}-CO-O- (worin b wie in Anspruch 1 definiert ist).

10. Kontrastmittel nach Anspruch 7, wobei das Emugliermittel ein Extended Polymer ist, umfassend einen hydrophilen Methoxy-terminierten Polyethylenglykolblock, acyliert mit einer hydrophoben Einheit, umfassend eine Kette aus zwei oder mehreren Fettsäuren.

11. Kontrastmittel nach Anspruch 10, wobei die hydrophobe Einheit eine Acyloxyacylgruppe ist.

12. Kontrastmittel nach Anspruch 11, wobei die hydrophobe Einheit 16-Hexadecanoyloxyhexadecanoyl ist.

13. Verfahren zur Herstellung eines Kontrastmittels nach Anspruch 1, umfassend die Einbringung eines Gases in ein bioabbaubares Polymer, umfassend wiederkehrende Einheiten der Formel (II) wie in Anspruch 1 definiert, um so Polymermikroteilchen und/oder Mikroballoons zu bilden.

14. Verfahren nach Anspruch 13, umfassend das Emulgieren einer Lösung des Polymeren in einem wasserunmischbaren, organischen Lösungsmittel in einer wäßrigen Phase und danach Entfernen mindestens der organischen Phase unter einer Atmosphäre des Gases, das einzubringen ist.

15. Verfahren nach Anspruch 14, wobei die wäßrige Phase ein Emulgiermittel, wie in mindestens einem der Ansprüche 5-12 definiert, enthält,

16. Verfahren nach Anspruch 14 oder Anspruch 15, wobei die Emulsion vor der Phasenentfernung filtriert und/oder extrudiert wird.

17. Bioabbaubare Polymere, bestehend aus wiederkehrenden Einheiten der Formel (II) wie in Anspruch 1 definiert.

18. Bioabbaubare Polymere nach Anspruch 17, wobei a eine ganze Zahl im Bereich von 13-17 bedeutet und b eine ganze Zahl im Bereich von 3-6, bedeutet.

19. Bioabbaubare Polymere nach Anspruch 17, wobei a 15 ist und b 4 ist.

20. Chirurgisches Implantat, Weichgewebeprothese, Schwamm, Film bzw. Folie, Wundverband, flexibles Blatt, Behälter, medizinische oder landwirtschaftliche Formulierung mit verzögerter Freisetzung, teilchenförmiges Abbildungsmittel oder Weichmacher, umfassend ein Polymer nach mindestens einem der Ansprüche 17-19.

21. Verfahren zur Herstellung eines Polymeren nach Anspruch 17, umfassend das Umsetzen eines reaktiven Derivats einer Disäure der Formel
HOOC.(CH₂)_{b}.COOH
(worin b wie in Anspruch 1 definiert ist) mit einem Diol der Formel
HO.(CH₂)ₐ.CO.O.CH(CH₃).O.CO.(CH₂)ₐ.OH
(worin a wie in Anspruch 1 definiert ist).

## Revendications

1. Agent de contraste comprenant des microparticules et/ou des microballons de polymère contenant du gaz **caractérisé en ce que** le polymère est un polymère biodégradable constitué de motifs répétitifs de formule(II) 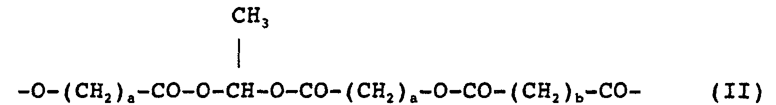 (dans laquelle a représente un nombre entier dans la gamme de 9 à 19 et b représente un nombre entier dans la gamme de 1 à 8).

2. Agent de contraste selon la revendication 1, dans lequel a représente un nombre entier dans la gamme de 13 à 17 et b représente un nombre entier dans la gamme de 3 à 6.

3. Agent de contraste selon la revendication 1, dans lequel a est égal à 15 et b est égal à 4.

4. Agent de contraste selon l'une quelconque des revendications 1 à 3, contenant un ou plusieurs additifs choisis parmi des agents émulsionnants, des agents de revêtement, des plastifiants, des agents d'étof fement, des cryoprotecteurs et des antioxydants.

5. Agent de contraste selon la revendication 4, contenant un agent émulsionnant choisi parmi des acides gras, des esters de glucides d'acides gras, des esters triglycérides d'acides gras, des protéines, des phospholipides, des polysaccharides et des polymères tensioactifs.

6. Agent de contraste selon la revendication 5, dans lequel l'agent émulsionnant est de l'albumine de sérum humain, de l'amidon modifié ou de la gélatine.

7. Agent de contraste selon la revendication 5, dans lequel l'agent émulsionnant est du poly(alcool vinylique) ou un copolymère séquencé/polymère prolongé.

8. Agent de contraste selon la revendication 7, dans lequel le copolymère séquencé/polymère prolongé contient des motifs issus du polyéthylèneglycol comme séquences hydrophiles et des motifs de formule (III)
-R^{a}-(CH₂)ₐ-CO-O-CH(CH₃)-O-CO-(CH₂)ₐ-R^{b}- (III)
(dans laquelle a est tel que défini dans la revendication 1 et R^{a} et R^{b} représentent chacun des liaisons de valence ou des groupes de liaison) dans la partie hydrophobe comme fragment de prolongation ou comme fragment dans une séquence oligomérique ou polymérique.

9. Agent de contraste selon la revendication 8, dans lequel R^{a} et R^{b} sont choisis chacun parmi des groupes carbonyle et des groupes de formule -O-CO-(CH₂)_{b}-CO-O- (dans laquelle b est tel que défini dans la revendication 1).

10. Agent de contraste selon la revendication 7, dans lequel l'agent émulsionnant est un polymère prolongé comprenant une séquence hydrophile de polyéthylèneglycol à terminaison méthoxy, acylée avec un fragment hydrophobe comprenant une chaîne de deux acides gras ou plus.

11. Agent de contraste selon la revendication 10, dans lequel le fragment hydrophobe est un groupe acyloxyacyle.

12. Agent de contraste selon la revendication 11, dans lequel le fragment hydrophobe est un fragment 16-hexadécanoyloxyhexadécanoyle.

13. Procédé de préparation d'un agent de contraste selon la revendication 1, qui comprend l'incorporation d'un gaz dans un polymère biodégradable comprenant des motifs répétitifs de formule (II) tels que définis dans la revendication 1, de manière à former des microparticules et/ou dés microballons de polymère.

14. Procédé selon la revendication 13, qui comprend la mise en émulsion d'une solution de polymère dans un solvant organique non miscible à l'eau dans une phase aqueuse et l'élimination ultérieure d'au moins la phase organique sous une atmosphère du gaz que l'on doit incorporer.

15. Procédé selon la revendication 14, dans lequel la phase aqueuse contient un agent émulsionnant tel que définis dans l'une quelconque des revendications 5 à 12.

16. Procédé selon la revendication 14 ou la revendication 15, dans lequel on filtre et/ou on extrude l'émulsion avant l'élimination de la phase.

17. Polymères biodégradables constitués de motifs répétitifs de formule (II) tels que définis dans la revendication 1.

18. Polymères biodégradables selon la revendication 17, dans lesquels a représente un nombre entier dans la gamme de 13 à 17 et b représente un nombre entier dans la gamme de 3 à 6.

19. Polymères biodégradables selon la revendication 17, dans lesquels a est égal à 15 et b est égal à 4.

20. Implant chirurgical, prothèse de tissu mou, éponge, film, pansement pour plaie, feuille souple, récipient, composition médicale ou agricole à libération retardée, agent d'imagerie particulaire ou plastifiant comprenant un polymère selon l'une quelconque des revendications 17 à 19.

21. Procédé de préparation d'un polymère selon la revendication 17, qui comprend la réaction d'un dérivé réactif d'un diacide de formule
HOOC.(CH₂)_{b}.COOH
(dans laquelle b est tel que défini dans la revendication 1) avec un diol de formule
HO.(CH₂)ₐ.CO.O.CH(CH₃).O.CO.(CH₂)ₐ.OH
(dans laquelle a est tel que défini dans la revendication 1).
